# EUROPÄISCHE PATENTSCHRIFT

(11) **EP 1 268 522 B1**
(45) Veröffentlichungstag und Bekanntmachung des Hinweises auf die Patenterteilung: **17.09.2008**
(21) Anmeldenummer: 01933682.5
(22) Anmeldetag: 12.03.2001
(51) Int. Cl.: C07K 7/23

(54) **LH-RH-ANTAGONISTEN, DEREN HERSTELLUNG UND VERWENDUNG ALS ARZNEIMITTEL**
NOVEL LHRH-ANTAGONISTS, PRODUCTION AND USE THEREOF AS MEDICAMENT
NOUVEAUX ANTAGONISTES DE LA LHRH, SA PRODUCTION ET SON UTILISATION COMME MEDICAMENT

(30) Priorität: 14.03.2000 US 525007
(43) Veröffentlichungstag der Anmeldung: 02.01.2003
(73) Patentinhaber: AEterna Zentaris GmbH, 60314 Frankfurt (DE)
(72) Erfinder: BERND, Michael, 60316 Frankfurt (DE); KUTSCHER, Bernhard, 63477 Maintal (DE); GÜNTHER, Eckhard, 63477 Maintal (DE); ROMEIS, Peter, 63571 Gelnhausen (DE); REISSMANN, Thomas, 60437 Frankfurt (DE); BECKERS, Thomas, 78464 Konstanz (DE)
(86) Internationale Anmeldenummer: PCT/EP2001/002719
(87) Internationale Veröffentlichungsnummer: WO 2001/068676

(56) Entgegenhaltungen:
- EP-A- 0 413 209
- WO-A-00/55190
- WO-A-95/00168
- WO-A-97/19953
- HAVIV F ET AL: "The Effect of NMeTyr5 Substitution in Luteinizing Hormone-Releasing Hormone Antagonists" JOURNAL OF MEDICINAL CHEMISTRY, AMERICAN CHEMICAL SOCIETY. WASHINGTON, US, Nr. 36, 1993, Seiten 928-933, XP002194666 ISSN: 0022-2623
- BRAUN K. ET AL: "Stability of the LHRH antagonists against proteolytic enzymes and idenfication of degradation products by mass spectrometry" PHARMAZIE, Bd. 56, Nr. 1, Januar 2001 (2001-01), Seiten 45-49, XP002194667

## Beschreibung

Die Erfindung betrifft LHRH-Antagonisten mit verbesserten Löslichkeitseigenschaften, Verfahren zur Herstellung dieser Verbindungen, Arzneimittel, in denen diese Verbindungen enthalten sind, sowie die Verwendung der Arzneimittel zur Behandlung hormonabhängiger Tumore und hormonbeeinflußter nicht-maligner Erkrankungen wie benigner Prostatahyperplasie (BPH) und Endometriose.

Die zur Definierung der Peptide verwendete Nomenklatur stimmt mit jener durch die IUPAC-IUB-Kommission über Biochemische Nomenklatur erläuterten Nomenklatur überein (European J.Biochem. 1984, 138, 9-37), worin in Übereinstimmung mit der herkömmlichen Darstellung die Aminogruppen beim N-Terminus nach links erscheinen und die Carboxylgruppe beim C-Terminus nach rechts. Die LH-RH-Antagonisten wie die erfindungsgemäßen Peptide umfassen in der Natur vorkommende und synthetische Aminosäuren, wobei erstere Ala, Val, Leu, Ile, Ser, Thr, Lys, Arg, Asp, Asn, Glu, Gln, Cys, Met, Phe, Tyr, Pro, Trp und His umfassen. Die Abkürzungen für die einzelnen Aminosäurereste beruhen auf den Trivialnamen der Aminosäuren und sind Ala=Alanin, Arg=Arginin, Gly=Glycin, Leu=Leucin, Lys=Lysin, Pal(3)=3-(3-Pyridyl)alanin, Nal(2)=3-(2-Naphthyl)alanin, Phe=Phenylalanin, Cpa=4-Chlorphenylalanin, Pro=Prolin, Ser=Serin, Thr=Threonin, Trp=Tryptophan, Tyr=Tyrosin und Sar=Sarkosin. Alle hier beschriebenen Aminosäuren stammen aus der L-Serie, wenn nicht anders erwähnt. Beispielsweise ist D-Nal(2) die Abkürzung für 3-(2-Naphthyl)-D-Alanin und Ser die Abkürzung für L-Serin. Substitutionen an der ε-Aminogruppe in der Seitenkette von Lysin sind durch einen hinter Lys in Klammern gesetzten Ausdruck, gegebenenfalls in Form einer Abkürzung, dargestellt.

Andere verwendete Abkürzungen sind:
- Ac: Acetyl

- B: 4-(4-Amidino-phenyl)-amino-1,4-dioxo-butyl
- Boc: tert. Butyloxycarbonyl
- Bop: Benzotriazol-1-oxy-tris-(dimethylamino)-phosphonium-hexafluorophosphat
- DCC: Dicyclohexylcarbodiimid
- DCM: Dichlormethan
- Ddz: Dimethoxyphenyl-dimethylmethylenoxy-carbonyl (Dimethoxy-dimethyl-Z)
- DIC: Diisopropylcarbodiimid
- DIPEA: N,N-Diisopropylethylamin
- DMF: Dimethylformamid
- Fmoc: Fluorenylmethyloxycarbonyl
- HF: Flußsäure
- HOBt: 1-Hydroxybenzotriazol
- HPLC: Hochdruckflüssigkeitschromatographie
- Me: Methyl
- TFA: Trifluoressigsäure
- Z: Benzyloxycarbonyl

Die erfindungsgemäßen Peptide stellen Analoge des das luteinisierende Hormon freisetzenden Hormons (LH-RH) dar, das die folgende Struktur aufweist: p-Glu-His-Trp-Ser-Tyr-Gly-Leu-Arg-Pro-Gly-NH₂, [LH-RH, Gonadorelin].

Während mehr als 20 Jahren haben Forscher nach selektiv potenten Antagonisten des LH-RH-Dekapeptids [M.Karten und J.E.Rivier, Endocrine Reviews 7, 44-66 (1986)] gesucht. Das hohe Interesse an solchen Antagonisten ist in ihrer Nützlichkeit im Bereich der Endokrinologie, Gynäkologie, Schwangerschaftsverhütung und Krebs begründet. Eine große Anzahl von Verbindungen sind als potentielle LH-RH-Antagonisten hergestellt worden. Die interessantesten Verbindungen, die bis heute gefunden wurden, sind jene Verbindungen, deren Strukturen eine Modifizierung der LH-RH-Struktur darstellen.

Die erste Serie von potenten Antagonisten wurde durch die Einführung von aromatischen Aminosäureresten in den Positionen 1, 2, 3 und 6 oder 2, 3 und 6 erhalten. Die übliche Schreibweise der Verbindungen sieht wie folgt aus: Es werden zunächst die Aminosäuren angegeben, die in der Peptidkette von LH-RH an die Stelle der ursprünglich vorhandenen Aminosäuren getreten sind, wobei die Positionen, an denen der Austausch stattfand, durch hochgestellte Ziffern gekennzeichnet werden. Weiterhin wird durch die nachgestellte Bezeichnung "LH-RH" zum Ausdruck gebracht, daß es sich um LH-RH-Analoge handelt, an denen der Austausch stattfand.

Bekannte Antagonisten sind:
[Ac-D-Cpa^{1,2}, D-Trp^{3,6}] LH-RH (D,H.Coy et al., In: Gross, E. and Meienhofer, J. (Eds) Peptides; Proceedings of the 6th American Peptide Symposium, S.775-779, Pierce Chem.Co., Rockville III. (1979):
[Ac-Pro¹ ,D-Cpa², D-Nal(2)^{3,6}] LH-RH (US-Patent Nr. 4.419.347) und
[Ac- Pro¹, D-Cpa² ,D-Trp^{3,6}] LH-RH (J.L.Pineda, et al., J. Clin. Endocrinol. Metab. 56, 420, 1983).

Um die Wirkung von Antagonisten zu verbessern, wurden später basische Aminosäuren, zum Beispiel D-Arg, in der 6-Stellung eingeführt. Zum Beispiel [Ac-D-Cpa^{1,2}, D-Trp³, D-Arg⁶, D-Ala¹⁰] LH-RH (ORG-30276) (D.H.Coy, et al., Endocrinology 100, 1445, 1982); und [Ac-D-Nal(2)1, D-Phe(4-F)², D-Trp³, D-Arg⁶] LH-RH (ORF 18260) (J.E. Rivier et al., in: Vickery B.H. Nestor, Jr. J.J., Hafez, E.S.E (Eds). LHRH and its Analogs, S.11-22 MTP Press, Lancaster, UK 1984).

Weitere potente LH-RH-Antagonisten sind in WO 92/19651, WO 94/19370, WO 92/17025, WO 94/14841, WO 94/13313, US-A 5,300,492, US-A 5,140,009, EP 0 413 209 A1 und DE 195 44 212 A1 beschrieben.

Letztere offenbart Verbindungen mit einem modifizierten Ornithin- oder Lysin-Baustein in Position 6, welche der folgenden Formel entsprechen:

Ac-D-Nal(2)¹-D-Cpa²-D-Pal(3)³-Ser⁴-Tyr⁵-D-Xxx⁶-Leu⁷-Arg⁸-Pro⁹-D-Ala¹⁰-NH₂,

worin D-Xxx eine Aminosäuregruppe der allgemeinen Formel (VI) 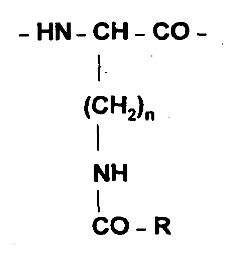 darstellt.

Weitere bekannte LH-RH-Antagonisten sind Antarelix, Ganirelix und Cetrorelix.

### Antarelix ® (INN: Teverelix):

Ac-D-Nal(2)¹-D-Cpa²-D-Pal(3)³-Ser⁴-Tyr⁵-D-Hci⁶-Leu⁷-Lys(iPr)⁸-Pro⁹-D-Ala¹⁰-NH₂

### Ganirelix:

Ac-D-Nal(2)¹-D-Cpa²-D-Pal(3)³-Ser⁴-Tyr⁵-D-hArg(Et)₂⁶-Leu⁷-hArg(Et)₂⁸-Pro⁹-D-Ala¹⁰-NH₂

### Cetrorelix:

Ac-D-Nal(2)¹-D-Cpa²-D-Pal(3)³-Ser⁴-Tyr⁵-D-Cit⁶-Leu⁷-Arg⁸-Pro⁹-D-Ala¹⁰-NH₂

Ein weiterer, nächstliegender Stand der Technik ist das Dokument WO 00/55190 A1. Das Patent beschreibt Peptide, die N-methylierte Aminosäurebausteine enthalten und eine verbesserte Wasserlöslichkeit aufweisen.

Als weiterer Stand der Technik wird auch das Dokument Haviv F. et al., "The Effect of NMe Tyr5 Substitution in Luteinizing Hormone-Releasing Hormone Antagonists", J. Med. Chem. 1993, 36, 928-933 genannt. Das Dokument offenbart LHRH-Antagonisten, die in Position 5 der Decapeptide eine N-Me-Tyr Aminosäure enthalten. Beschrieben wird der Effekt einer verbesserten Wasserlöslichkeit und proteolytischen Enzymresistenz.

Ziel der Erfindung ist, neue LH-RH-Antagonisten zu schaffen, die eine erhöhte enzymatische Stabilität und signifikant verbesserte Wasserlöslichkeit aufweisen.

Diese Aufgabe wird durch Verbindungen gelöst,
deren Umfang gekennzeichnet wird durch folgende Verbindungen:

Ac-D-Nal(2)¹-D-Cpa²-D-Pal(3)³-Ser⁴-Tyr⁵-D-Lys(B)⁶-Nle⁷-Arg⁸-Pro⁹-Sar¹⁰-NH₂

Ac-D-Nal(2)¹-D-Cpa²-D-Pal(3)³-Ser⁴-N-Me-Tyr⁵-D-Lys(B)⁶-Nle⁷-Arg⁸-Pro⁹-D-Ala¹⁰-NH₂

Ac-D-Nal(2)¹-D-Cpa²-D-Pal(3)³-Ser⁴-N-Me-Tyr⁵-D-Lys(B)⁶-Nle⁷-Lys(iPr)⁸-Pro⁹-D-Ala¹⁰-NH₂

Ac-D-Nal(2)¹-D-Cpa²-D-Pal(3)³-Ser⁴-N-Me-Tyr⁵-D-Lys(B)⁶-Nle⁷-Lys(iPr)⁸-Pro⁹-D-Ala¹⁰-NH₂

Gemäß einem weiteren Aspekt der Erfindung liegen die erfindungsgemäßen Verbindungen als Acetat-, Trifluoracetat- oder Embonat-Salz vor.

Die Erfindung bezieht sich auch auf die erfindungsgemäßen Verbindungen zur Verwendung als Arzneimittel bzw. pharmazeutische Zubereitungen.

Gemäß einem weiteren Aspekt der Erfindung werden pharmazeutische Zubereitungen, umfassend mindestens einer der erfindungsgemäßen Verbindungen und übliche Träger- und Hilfsstoffe, bereitgestellt.

Gemäß einem weiteren Aspekt der Erfindung wird ein Verfahren zur Herstellung der erfindungsgemäßen Verbindungen der allgemeinen Formel I

A-Xxx¹-Xxx²-Xxx³-Xxx⁴-Xxx⁵-Xxx⁶-Xxx⁷-Xxx⁸-Xxx⁹-Xxx¹⁰-NH₂ (I)

mit A in der Bedeutung Acetyl
bereitgestellt, bei dem Fragmente aus mit geeigneten Schutzgruppen versehenen Bausteinen Xxx^{m}, bei denen m eine ganze Zahl von 1 bis 10 bedeutet und Xxx¹ acetyliert ist, an einer Festphase oder in Lösung nach üblichen Verfahren aufgebaut werden, anschließend die Fragmente an einer Festphase durch Segmentkupplung verbunden werden und nach Abschluss der Kupplung die Verbindungen der allgemeinen Formel I mit üblichen Verfahren unter Amidierung am Baustein Xxx¹⁰ von der Festphase abgespalten werden.
Gemäß einem weiteren Aspekt der Erfindung wird die Verwendung der erfindungsgemäßen Verbindungen zur Herstellung von Arzneimitteln zur Behandlung hormonabhängiger Tumore, insbesondere Prostatacarcinom oder Brustkrebs, sowie für nicht-maligne Indikationen, deren Behandlung eine LH-RH-Hormonsuppression erfordert, bereitgestellt.
Gemäß einem weiteren Aspekt der Erfindung wird ein Verfahren zur Herstellung von pharmazeutischen Zubereitungen bereitgestellt, wobei man mindestens eine Verbindung gemäß einem der Ansprüche 1 und 2 mit den üblichen Träger- und Hilfsstoffen vermischt und als Arzneimittel konfektioniert.

Die erfindungsgemäßen Verbindungen können zur Behandlung hormonabhängiger Tumore, insbesondere Prostatacarcinom, Brustkrebs oder Uterusmyom, sowie für nicht-maligne Indikationen, deren Behandlung eine LH-RH-Hormonsuppression erfordert, wie Endometriose oder benigne Prostatahyperplasie (BPH) verwendet werden. Weiterhin können sie zur Behandlung von Fruchtbarkeitsstörungen bei der Frau oder beim Mann eingesetzt werden, beispielsweise zur kontrollierten ovariellen Superstimulation im Rahmen der künstlichen Befruchtung (in-vitro Fertilisation). Dazu werden sie üblicherweise nach an sich bekannten Verfahren mit herkömmlichen Träger- und Hilfsstoffen vermischt und als Arzneimittel konfektioniert.
Die Synthese von Verbindungen gemäß Formel (I) kann sowohl entweder durch klassische Fragmentkondensation oder per Festphasensynthese nach Merrifield mit aufeinander abfolgendem Aufbau unter Verwendung von in der Seitenkette bereits mit der Carbonsäure der allgemeinen Formel R¹-COOH acyliertem D-Lysin als auch durch Umsetzung eines Decapeptidbausteins mit den entsprechenden Carbonsäuren durch Amid-Verknüpfung in der Seitenkette von D-Lysin⁶ erfolgen. Demnach kann die Einführung der R¹-CO-Gruppe an drei verschiedenen Stellen des Verfahrens vorgenommen werden: vor der Kondensation der Einzelbausteine zum Peptid, nach dem Einbau von Lysin oder Ornithin in der Peptidkette, aber vor der Kondensation des nächstfolgenden Bausteins oder nach Kondensation aller Bausteine.
Die Verbindungen der Formel (I) werden nach den bekannten Methoden synthetisiert, wie zum Beispiel durch reine Festphasentechnik, teilweise Festphasentechnik (sogenannte Fragmentkondensation) oder durch die klassischen Lösungskupplungen (siehe M.Bodanszky, "Principles of Peptide Synthesis", Springer Verlag 1984).
Zum Beispiel sind die Methoden der Festphasensynthese im Lehrbuch "Solid Phase Peptide Synthesis" J.M.Stewart and J.D.Young, Pierce Chem.Company, Rockford, III, 1984, und in G.Barany and R.B.Merrifield "The Peptides", Ch.1, S.1-285, 1979, Academic Press Inc. beschrieben. Klassische Lösungssynthesen sind ausführlich in der Behandlung "Methoden der Organischen Chemie (Houben-Weyl), Synthese von Peptiden" E.Wünsch (Herausgeber) 1974, Georg Thieme Verlag, Stuttgart, BRD, beschrieben.
Der stufenweise Aufbau erfolgt zum Beispiel, indem man zunächst die Carboxyterminale Aminosäure, deren α-ständige Aminogruppe geschützt ist, an einen hierfür üblichen unlöslichen Träger kovalent bindet, die α-Amino-Schutzgruppe dieser Aminosäure abspaltet, an die so erhaltene freie Aminogruppe die nächste geschützte Aminosäure über ihre Carboxy-Gruppe bindet, und in dieser Weise Schritt für Schritt die übrigen Aminosäuren des zu synthetisierenden Peptids in der richtigen Reihenfolge verknüpft, und nach Verknüpfung aller Aminosäuren das fertige Peptid vom Träger abspaltet und gegebenenfalls weitere vorhandene Seitenfunktions-Schutzgruppen abspaltet. Die stufenweise Kondensation erfolgt durch Synthese aus den entsprechenden, in üblicher Weise geschützten Aminosäuren in herkömmlicher Weise.

Die Verknüpfung der einzelnen Aminosäuren miteinander erfolgt nach den hierfür üblichen Methoden, insbesondere kommen in Frage:
**•** Methode der symmetrischen Anhydride in Gegenwart von Dicyclohexylcarbodiimid oder Diisopropylcarbodiimid (DCC, DIC)
**•** Carbodiimid-Methode allgemein
**•** Carbodiimid-Hydroxybenzotriazol-Methode
(siehe The Peptides, Volume 2, Ed. E. Gross and J. Meienhofer).

Bei der Fragmentkupplung verwendet man vorzugsweise die ohne Racemisierung verlaufende Azidkupplung oder die DCC-1-Hydroxybenzotriazol-beziehungsweise DCC-3-Hydroxy-4-oxo-3,4-dihydro-1,2,3-benzotriazin-Methode. Man kann auch aktivierte Ester von Fragmenten einsetzen.

Für die stufenweise Kondensation von Aminosäuren eignen sich besonders gut aktivierte Ester von N-geschützten Aminosäuren, wie zum Beispiel N-Hydroxysuccinimidester oder 2,4,5-Trichlorphenylester. Die Aminolyse läßt sich sehr gut durch N-Hydroxyverbindungen, die in etwa die Acidität der Essigsäure besitzen, wie zum Beispiel 1-Hydroxybenzotriazol, katalysieren.

Als intermediäre Aminoschutzgruppen bieten sich abhydrierende Gruppen, wie zum Beispiel der Benzyloxycarbonylrest (= Z-Rest) oder schwach sauer abspaltbare Gruppen an. Als Schutzgruppen für die α-ständigen Aminogruppen kommen zum Beispiel in Frage:
tertiäre Butyloxycarbonylgruppen, Fluorenylmethyloxycarbonylgruppen, Carbobenzoxygruppen beziehungsweise Carbobenzthiogruppen (gegebenenfalls jeweils mit p-Brom oder p-Nitro-benzylrest), die Trifluoracetylgruppe, der Phthalylrest, die o-Nitrophenoxyacetylgruppe, die Tritylgruppe, die p-Toluolsulfonylgruppe, die Benzylgruppe, im Benzolkern substituierte Benzylreste (p-Brom- oder p-Nitro-benzylrest) und der α-Phenyl-ethylrest. Hierzu wird auch auf Jesse P. Greenstein und Milton Winitz, Chemistry of Amino Acids, New York 1961, John Wiley and Sons, Inc., Volume 2, beispielsweise Seite 883 und folgende, "Principles of Peptide Synthesis", Springer Verlag 1984, "Solid Phase Peptide Synthesis" J.M.Stewart and J.D.Young, Pierce Chem.Company, Rockford, III, 1984, G.Barany and R.B.Merrifield "The Peptides", Ch.1, S.1-285, 1979, Academic Press Inc. sowie The Peptides, Volume 2, Ed.E. Gross and J. Maienhofer, Academic Press, New York, verwiesen. Diese Schutzgruppen kommen grundsätzlich auch für den Schutz von weiteren funktionellen Seitengruppen (OH-Gruppen, NH₂-Gruppen) der entsprechenden Aminosäuren in Frage.

Vorhandene Hydroxygruppen (Serin, Threonin) werden vorzugsweise durch Benzylgruppen und ähnliche Gruppen geschützt. Weitere nicht α-ständige Aminogruppen (zum Beispiel Aminogruppen in ω-Stellung, Guanidinogruppe des Arginins) werden vorzugsweise orthogonal geschützt.

Die einzelnen Aminosäurebausteine sind, ausgenommen durch die R¹-CO-Gruppe modifiziertes Lysin, käuflich erhältlich.

Ein möglicher Ablauf des Verfahrens zur Herstellung von durch R¹-CO-Gruppe modifiziertes Lysin ist wie folgt:
1. Die α-Carbonsäuregruppe wird geeignet geschützt, beispielsweise durch Veresterung.
2. Die ε-Aminogruppe wird geschützt, beispielsweise mit der Z-Gruppe,
3. Die α-Aminogruppe wird derart geschützt (z.B. Boc-Gruppe), daß sich eine Selektivität bezüglich der späteren Abspaltung der Aminoschutzgruppen ergibt.
4. Die Z-Gruppe an der ε-Aminogruppe wird abgespalten.
5. An der ε-Aminogruppe wird die gewünschte Gruppe R¹-CO- eingeführt.
6. Die Schutzgruppe an der α-Aminogruppe wird abgespalten,
7. Die α-Aminogruppe wird gegebenenfalls reversibel derivatisiert, z.B. mit der Z-Gruppe.

Für die Einführung der R¹-CO-Gruppe durch Umsetzen der Aminogruppe des Lysins mit der entsprechenden Carbonsäure bzw. Carbonsäurederivat kommen grundsätzlich die gleichen Verfahren wie oben für die Verknüpfung der Aminosäuren beschriebenen in Frage. Besonders bevorzugt ist jedoch die Kondensation unter Verwendung von Carbodiimid, beispielsweise 1-Ethyl-3-(3-dimethylaminopropyl)-carbodiimid, und 1-Hydroxybenzotriazol.

Die Reaktion zur Verknüpfung von Aminosäuren findet in einem hierfür üblichen indifferenten Lösungs- oder Suspensionsmittel (zum Beispiel Dichlormethan) statt, wobei gegebenenfalls zur Verbesserung der Löslichkeit Dimethylformamid zugesetzt werden kann.

Als synthetisches Trägermaterial kommen unlösliche Polymere in Frage, zum Beispiel in organischem Lösungsmittel quellbares Polystyrolharz in Perlenform (beispielsweise ein Copolymerisat aus Polystyrol und 1 % Divinylbenzol). Der Aufbau eines geschützten Decapeptidamids an einem Methyl-benzhydrylamin-Harz (MBHA-Harz, d.h. mit Methyl-benzhydrylamin-Gruppen versehenes Polystyrolharz), welches die gewünschte C-terminale Amidfunktion des Peptids nach HF-Spaltung vom Träger ergibt, kann gemäß folgendem Fließdiagramm durchgeführt werden:

### Fließdiagramm

Peptid-Syntheseprotokoll unter Verwendung von Boc-geschützten Aminosäuren

| Stufe | Funktion | Lösungsmittel/Reagenz (v/v) | Zeit |
|---|---|---|---|
| 1 | Waschen | Methanol | 2 x 2 min |
| 2 | Waschen | DCM | 3 x 3 min |
| 3 | Abspaltung | DCM/TFA (1:1) | 1 x 30 min |
| 4 | Waschen | Isopropanol | 2 x 2 min |
| 5 | Waschen | Methanol | 2 x 2 min |
| 6 | Waschen | DCM | 2 x 3 min |
| 7 | Neutralisation | DCM/DIPEA (9:1) | 3 x 5 min |
| 8 | Waschen . | Methanol | 2 x 2 min |
| 9 | Waschen | DCM | 3 x 3 min |
| 10 | STOP | Zugabe der Boc-As in DCM + DIC + HOBt | |
| 11 | Kupplung | DCM, ggf. DCM/DCF | ca. 90 min |
| 12 | Waschen | Methanol | 3 x 2 min |
| 13 | Waschen | DCM | 2 x 3 min |

Die Nα-Boc-geschützten Aminosäuren werden üblicherweise in dreifachem molaren Überschuß in Gegenwart von Diisopropylcarbodiimid (DIC) und 1-Hydroxybenzotriazol (HOBt) in CH₂Cl₂/DMF innerhalb von 90 min. gekuppelt, und die Boc-Schutzgruppe durch halbstündige Einwirkung von 50% Trifluoressigsäure (TFA) in CH₂Cl₂ abgespalten. Zur Kontrolle des vollständigen Umsatzes kann der Chloraniltest nach Christensen und der Kaiser'sche Ninhydrintest dienen. Reste freier Aminofunktionen werden durch Acetylierung in fünffachem Überschuß an Acetylimidazol in CH₂Cl₂ blockiert. Die Abfolge der Reaktionsschritte des Peptidaufbaus am Harz ergibt sich aus dem Fließdiagramm. Zur Abspaltung der harzgebundenen Peptide wird das jeweilige Endprodukt der Festphasensynthese im Vakuum über P₂O₅ getrocknet und in 500fachem Überschuß an HF/Anisol 10:1/V:V 60 min. bei 0°C behandelt.

Nach Abdestillation von HF und Anisol im Vakuum fallen die Peptide durch Ausrühren mit wasserfreiem Ethylether als weiße Feststoffe an, die Abtrennung von mit anfallendem polymeren Träger erfolgt durch Auswaschen mit 50%iger wäßriger Essigsäure. Durch schonendes Einengen der essigsauren Lösungen im Vakuum können die jeweiligen Peptide als hochviskose Öle erhalten werden, welche sich nach Zugabe von abs.Ether in der Kälte in weiße Feststoffe umwandeln.

Die weitere Aufreinigung erfolgt durch Routinemethoden der präparativen Hochdruck-Flüssigskeitschromatographie (HPLC).

Das Überführen der Peptide in ihre Säureadditionssalze kann durch Umsetzen derselben mit Säuren in an sich bekannter Weise bewerkstelligt werden. Umgekehrt können freie Peptide durch Umsetzen ihrer Säureadditionssalze mit Basen erhalten werden. Peptidembonate können durch Umsetzung von Trifluoressigsäuresalzen (TFA-Salzen) des Peptids mit freier Embonsäure (Pamoasäure) oder dem entsprechenden Dinatrium-Salz der Embonsäure dargestellt werden. Dazu wird das Peptid-TFA-Salz in wäßriger Lösung mit der Lösung von Dinatrium-embonat in polar-aprotischem Medium, bevorzugt Dimethylacetamid, versetzt und der sich bildende hellgelbe Niederschlag isoliert.

Die folgenden Beispiele dienen der Erläuterung der Erfindung.

### Beispiel 1 (D-68968):

### Ac-D-Nal(2)¹-D-Cpa²-D-Pal(3)³-Ser⁴-N-Me-Tyr⁵-D-Lys(B)⁶-Nle⁷-Arg⁸-Pro⁹-Sar¹⁰-NH₂

Die Synthese des Dekapeptids erfolgte am polymeren Träger mit einer Beladungsdichte von 0,55 mmol/g (Aminomethyl-substituiertes Harz, Fmoc-Schutz, Typ D-1675, Fa. Bachem). Lysin wurde als Fmoc-D-Lys(Boc)-OH gekuppelt, die Fmoc-Schutzgruppen mit 20% Piperidin/DMF abgespaltet. Nach gleichzeitiger Abspaltung aller Seitenkettenschutzgruppen und Ablösung vom polymeren Träger wurde das isolierte Rohpeptid mittels präparativer HPLC gereinigt. Nach Gefriertrocknung erhielt man 98,5%iges Dekapeptid.

Die Substitution am ε-Stickstoff von D-Lysin mit 4-(4-Aminophenyl)-amino-1,4-dioxo-buttersäure gelang mit PyBop in DMF unter Zusatz von DIPEA. Die Reinigung des isolierten Rohpeptids erfolgte mittels präparativer HPLC. Die anschließende Gefriertrocknung ergab ca. 99%iges Produkt (Trifluoracetat) der Summenformel C₈₂H₁₀₆ClN₁₉O₁₅ mit korrektem FAB-MS 1633 (M+H) (ber. 1631,78096)

### Beispiel 2 (D-68969):

### Ac-D-Nal(2)¹-D-Cpa²-D-Pal(3)³-Ser⁴-N-Me-Tyr⁵-D-Lys(B)⁶-Nle⁷-Arg⁸-Pro⁹-D-Ala¹⁰-NH₂

Die Synthese des Dekapeptids erfolgte am polymeren Träger mit einer Beladungsdichte von 0,55 mmol/g (Aminomethyl-substituiertes Harz, Fmoc-Schutz, Typ D-1675, Fa Bachem). Lysin wurde als Fmoc-D-Lys(Boc)-OH gekuppelt, die Fmoc-Schutzgruppen mit 20% Piperidin/DMF abgespaltet. Nach gleichzeitiger Abspaltung aller Seitenkettenschutzgruppen und Ablösung vom polymeren Träger wurde das isolierte Rohpeptid mit einem Gehalt ca. 71 % (HPLC) ohne Reinigung weiter umgesetzt.

Die Seitenketten-Substitution von D-Lysin mit 4-(4-Aminophenyl)-amino-1,4-dioxo-buttersäure erfolgte mit PyBop in DMF unter Zusatz von DIPEA. Das isolierte Rohpeptid wurde mittels präparativer HPLC gereinigt. Nach anschließender Gefriertrocknung erhielt man ein 98,8%iges Produkt

(Trifluoracetat) der Summenformel C₈₂H₁₀₆ClN₁₉O₁₅ mit korrektem FAB-MS 1633 (M+H) (ber. 1631,78096)

### Beispiel 3 (D-68971):

### Ac-D-Nal(2)¹-D-Cpa²-D-Pal(3)³-Ser⁴-N-Me-Tyr⁵-D-Lys(B)⁶-Nle⁷-Lys(iPr)⁸-Pro⁹-Sar¹⁰-NH₂

Die Synthese des Dekapeptids erfolgte am polymeren Träger mit einer Beladungsdichte von 0,55 mmol/g (Aminomethyl-substituiertes Harz, Fmoc-Schutz, Typ D-1675, Fa. Bachem). Lysin wurde als Fmoc-D-Lys(Boc)-OH gekuppelt, die Fmoc-Schutzgruppen mit 20% Piperidin/DMF abgespaltet. Nach gleichzeitiger Abspaltung aller Seitenkettenschutzgruppen und Ablösung vom polymeren Träger wurde das isolierte Rohpeptid (Gehalt ca. 59%, HPLC) mittels präparativer HPLC gereinigt. Nach Gefriertrocknung erhielt man 95%iges Dekapeptid.
Die Seitenketten-Substitution von D-Lysin mit 4-(4-Aminophenyl)-amino-1,4-dioxo-buttersäure erfolgte mit PyBop in DMF unter Zusatz von DIPEA. Das isolierte Rohpeptid wurde mittels präparativer HPLC gereinigt. Nach anschließender Gefriertrocknung erhielt man ein 96,6%iges Produkt (Trifluoracetat) der Summenformel C₈₅H₁₁₂CIN₁₇O₁₅ mit korrektem FAB-MS 1648 (M+H) (ber. 1645,8218)

### Beispiel 4 (D-68987)

### Ac-D-Nal(2)¹-D-Phe(4-Cl)²-D-Pal(3)³-Ser⁴-N-Me-Tyr⁵-D-Lys(B)⁶-Nle⁷-Lys(iPr)⁸-Pro⁹-D-Ala¹⁰-NH₂

Die Synthese des D-Lys-6-unsubstituierten Dekapeptids erfolgte an 9.09 g polymerem Träger mit einer Beladungsdichte von 0,55 mmol/g, Lysin⁶ wurde als Fmoc-D-Lys(Boc)-OH gekuppelt.

Nach Abspaltung vom Harz wurden 8.15 g Rohpeptid isoliert. Die Reinigung des Rohpeptids erfolgte mittels präparativer HPLC.

Die Seitenketten-Substitution von D-Lysin mit 4-(4-Aminophenyl)-amino-1,4-dioxo-buttersäure gelang mit PyBop in DMF, unter Zusatz von DIPEA. Das isolierte Rohpeptid wurde mittels präparativer HPLC gereinigt. Nach anschließender Gefriertrocknung erhielt man ein 94,6%iges Produkt (Trifluoracetat) der Summenformel C85 H112 Cl N17 015 mit entsprechendem FAB-MS: 1646.8 (M+H; berechnet: 1645.82).

### Untersuchungen zur biologischen Wirkung:

Die erfindungsgemäßen Verbindungen werden auf ihre Rezeptorbindung untersucht. Das Verfahren lehnt sich eng an das in Beckers et al., Eur. J. Biochem. 231, 535-543 (1995), beschriebene Verfahren an. Nach der oben offenbarten Synthese erhaltenes Cetrorelix wird mit [¹²⁵I] (Amersham; spezifische Aktivität 80.5Bq/fmol) unter Verwendung des IodoGen-Reagens (Pierce) iodiert. Das Reaktionsgemisch wird durch Umkehrphasen-Hochleistungs-Flüssigkeitschromatographie gereinigt, wobei mono-iodiertes Cetrorelix ohne unmarkiertes Peptid erhalten wird. Jeweils etwa 80% des
[¹²⁵I]-Cetrorelix und der nicht markierten erfindungsgemäßen Verbindung sind zur spezifischen Rezeptorassoziation geeignet.

Die erfindungsgemäßen Verbindungen können mit den folgenden Methoden 1 und 2 auf ihre In-vitro-Wirkung getestet werden, wobei die Bindungsaffinitäten im Bindungsassay mit [¹²⁵I]-Cetrorelix (Methode 1) und die funktionalen Aktivitäten mit Triptorelin als agonistischem Stimulus (Methode 2) bestimmt werden.

### Methode 1 (Bestimmung von K_{D} am Beispiel von Cetrorelix):

Rezeptorbindungs-Assay nach Beckers, T., Marheineke, K., Reiländer, H., Hilgard P. (1995) "Selection and characterization of mammalian cell lines with stable overexpression of human pituitary receptors for gonadoliberin (GnRH)" Eur. J. Biochem. 231, 535 - 543.

Zur Untersuchung der Rezeptorbindung wird Cetrorelix unter Verwendung des IodoGen-Reagens (Pierce) mit [¹²⁵I] (Amersham; 80.5Bq/fmol spezifische Aktivität) iodiert. Das Reaktionsgemisch wird durch Hochleistungs-Flüssigkeitschromatographie mit vertauschten Phasen gereinigt, wobei mono-iodiertes Cetrorelix ohne unmarkiertes Peptid erhalten wird. Etwa 80% des [¹²⁵I] Cetrorelix war zur spezifischen Rezeptorassoziation befähigt.

Der Rezeptorbindungs-Assay wird mit intakten Zellen unter physiologischen Bedingungen wie beschrieben (Beckers et al. 1995) durchgeführt. Subkonfluente Kultüren von stabil transfizierten LTK⁻-Zellen, die den humanen LHRH-Rezeptor exprimieren, werden durch Inkubation in NaCl/Pᵢ (137mM NaCl, 2.7mM KCI, 8.1 mM Na₂HPO₄, 11.47mM KH₂PO₄)/ 1mM EDTA abgetrennt und durch Zentrifugieren gesammelt. Das Zellpellet wird in Bindungspuffer (DMEM ohne H₂CO₃, mit 4.5g/l glucose, 10mM Hepes pH7.5, 0.5% (Masse/Volumen) BSA, 1g/l Bacitracin, 0.1g/l SBTI, 0.1% (Masse/Volumen) NaN₃) resuspendiert. Für Verdrängungs-Assays werden 0.25x10⁶ Zellen/100µl mit etwa 225pM des [¹²⁵I]-Cetrorelix (spezifische Aktivität 5 -10 x10⁵ dpm/pmol) und verschiedenen Konzentrationen von unmarkierter erfindungsgemäßer Verbindung als Kompetitor inkubiert. Die Zellsuspension in 100µl Bindungsmedium wird in 400µl Assayröhrchen über 200µl 84 Vol.-% Siliconöl (Merck Typ 550) / 16 Vol.-% Paraffinöl geschichtet. Nach Inkubation für 1 h bei 37°C unter langsamem, kontinuierlichem Schütteln werden die Zellen durch Zentrifugieren für 2min bei 9000rpm (Rotortyp HTA13.8; Heraeus Sepatec, Osterode/Germany) von dem Inkubationsmedium getrennt. Die Spitzen der Röhrchen, die das Zellpellet enthielten, werden abgeschnitten. Zellpellet und Überstand werden anschließend durch Zählung der γ-Strahlung analysiert. Die Menge an unspezifisch Gebundenem wird unter Einschluß von unmarkiertem Cetrorelix bei 1 µM Endkonzentration bestimmt und beträgt typischerweise ≤ 10% des gesamten Gebundenen. Die Analyse der Bindungsdaten wird mit dem EBDA/Ligand-Analyseprogramm (Biosoft V3.0) durchgeführt.

Cetrorelix weist einen K_{D}-Wert von 170 Pikomol pro Liter (pM) auf (Anzahl der unabhängig durchgeführten Versuche: 21).

Methode 2 (Funktioneller Assay zur Bestimmung der antagonistischen Wirksamkeit (IC₅₀-Wert)):
Der Assay wird mit nachstehend genannten Modifizierungen versehen so durchgeführt wie in Beckers, T., Reiländer, H., Hilgard, P. (1997) "Characterization of gonadotropin-releasing hormone analogs based on a sensitive cellular luciferase reporter gene assay", Analyt. Biochem. 251, 17 - 23 (Beckers et al. 1997) beschrieben. 10.000 Zellen pro Vertiefung, die den humanen LHRH-Rezeptor und ein Luciferase-Reportergen exprimieren, werden 24h in Mikrotiterplatten unter Verwendung von DMEM mit Zusätzen und 1 % (v:v) FCSᵢ kultiviert. Die Zellen werden anschließend 6h mit 1 nM [D-Trp⁶] LHRH stimuliert. Antagonistische erfindungsgemäße Verbindungen werden vor der Stimulierung zugegeben und die Zellen werden zum Schluß zur Quantifizierung der zellulären Luc-Activität lysiert. Die Berechnung der IC₅₀-Werte aus Dosis-Wirkungs-Kurven wird durch nicht-lineare Regressionsanalyse unter Verwendung des Hill-Modells (Programm EDX 2.0 von C. Grunwald, Arzneimittelwerk Dresden) durchgeführt.

Die Quantifizierung der Luc-Aktivität wird im wesentlichen wie beschrieben (Promega Technical Bulletins #101/161) unter Verwendung des jeweiligen Luciferase-Assaysystems (Promega E4030) in Duplikaten durchgeführt. Durch Zugabe von Coenzyme A (CoA) findet eine Oxidation von Luciferyl-CoA mit vorteilhafter Kinetik statt. Nach der Entfernung des Kulturmediums von der Mikrotiterplatte werden die Zellen durch Zugabe von 100µl Lysepuffer (25mM Tris-phosphate pH7.8, 2mM Dithiothreitol, 2mM 1,2-diaminocyclohexan-N,N,N',N'-tetra-essigsäure (CDTA), 10% (v:v) Glycerin, 1 % (v:v) Triton X-100) lysiert. Nach 15min Inkubation bei Raumtemperatur werden 10µl Zelllysat in eine für luminometrische Detektion geeignete weiße Mikrotiterplatte (Dynatech) überführt.

Die enzymatische Reaktion wird durch Zugabe von 50µl Assaypuffer (20mM Tricin pH7.8, 1.07mM (MgCO₃)₄Mg(OH)₂, 2.67mM MgSO₄, 0.1mM Ethylenediamin-tetraessigsäure (EDTA), 33.3mM Dithiothreitol, 270µM Coenzym A, 470µM Glühwürmchen(Photinus pyralis)-Luciferin, 530µM rATPNa₂) initiiert. Nach einer Minute wird für eine Gesamtzeit von einer Sekunde die Lumineszenz mit einer Signalhalbwertszeit von fünf Minuten unter Verwendung des EG&G Berthold MicroLumat LB 96 P bestimmt.

In Tabelle 2 sind physikalisch-chemische und in-vitro-Daten der erfindungsgemäßen Verbindungen zusammengefaßt. IC₅₀ steht für die funktionale Aktivität und pM bedeutet Pikomol pro Liter. Die Wasserlöslichkeit wurde nach dem unter Anmerkung 2) beschriebenen Verfahren bestimmt:

**Tabelle 2:**

| Verbindung | Wasserlöslichkeit² [mg/ml] | IC₅₀ [pM] |
|---|---|---|
| Cetrorelix | 0.002 | 198 (5) ¹ |
| Beispiel 1 (D-68968) | 1.03 | 1300 (1) ¹ |
| Beispiel 2 (D-68969) | 1.11 | 1400 (1) ¹ |
| Beispiel 3 (D-68971) | 1.36 | 4700 (1) ¹ |
| Beispiel 4 (D-68987) | 1.18 | 700 (1) ¹ |

| | | |
|---|---|---|
| Anmerkungen: 1) die in Klammern stehende Zahl gibt die Anzahl der voneinander unabhängigen Versuche an 2) Die Wasserlöslichkeit wurde gemäß der nachfolgend beschriebenen Methode bestimmt: | | |

### Löslichkeit nach Amtsblattmethode in Ringer Lösung:

Zur Bestimmung der Löslichkeit nach Amtsblattmethode wird die Prüfsubstanz im Überschuß mit einem inerten Trägermaterial wie z. B. Sand gemischt und in eine Glassäule (Volumengröße ca. 10 ml) gefüllt. In den Säulenboden wurde vorher ein Sieb aus Watte und Glasfaserfilter eingebaut. Das Substanz-Sand-Gemisch wird in 1.0 ml Lösungsmittel in dem die Löslichkeit bestimmt werden soll, 1 Stunde quellen gelassen. Anschließend werden 10 ml des Lösungsmittels in die Glassäule gefüllt. Mittels einer Schlauchpumpe wird die Lösung im Zyklus gepumpt. Diese Bestimmung wird bei Raumtemperatur (ca. 20°C) durchgeführt.
Die Löslichkeit wird bestimmt, wenn die Massenkonzentration von aufeinanderfolgender entnommenen Fraktionen konstant ist.
Die Bestimmung der Massenkonzentration wird mittels der nachstehend beschriebenen HPLC-Methode ermittelt.

### HPLC-Methode:

### Equipment

HPLC-system: Hewlett Packard 1100; Detector:Hewlett Packard DAD Series 1100

| | | | |
|---|---|---|---|
| Säule: | Säulenmaterial: | Nucleosil® 120-3 C₁₈ | |
| | Partikelgröße: | 3 µm | |
| | Säulengröße: | 125 x 4 mm | |
| | Hersteller: | | Macherey & Nagel |

| | | |
|---|---|---|
| Equipment parameters: | Injektionsvolumen: | 15 µl |
| Fluß: | 1.0 ml/min | |
| Ofen Temperatur: | 45° C | |
| Wellenlänge: | 226 nm | |
| Stop Zeit: | 15 min | |

55% Mobile Phase A : 970 ml Milli-Q-H2O, 30 ml Acetonitril und 1 ml Trifluoressigsäure werden gemischt. Der resultierende pH ist ca. 1.9.
45% Mobile Phase B : 300 ml Milli-Q-H2O, 700 ml Acetonitril und 1 ml Trifluoressigsäure werden gemischt. Der resultierende pH ist ca. 1.8.

Die Verabreichung der erfindungsgemäßen Verbindungen kann in unterschiedlicher, für peptidische Wirkstoffe geeigneter Form erfolgen. Geeignete Verabreichungen sind dem Fachmann wohlbekannt. Die Verabreichung kann beispielsweise durch Injektion erfolgen. Die Verabreichung kann beispielsweise parenteral durchgeführt werden. Bevorzugt sind dabei die subcutane (s.c.), intramuskuläre (i.m.), intravenöse (i.v.), bukkale (z.B. sublinguale) oder rekatale Verabreichung. Die i.s. und i.m. Verabreichung sind besonders bevorzugt.

Die erfindungsgemäßen Verbindungen sind zur Herstellung von unterschiedlichen Darreichungsformen geeignet, beispielsweise für Lyophilisate, Lösungen, oder Suspensionen. Geeignete Darreichungsformen und deren Herstellung sind dem Fachmann bekannt. Als geeignete Hilfs- und Gerüststoffe sind beispielsweise Hexite, wie Mannit, insbesondere D-Mannit, L-Mannit oder D, L-Mannit, Sorbit, wie D-Sorbit, D- oder L-Altrit, Idit, Glucit und Dulcit geeignet. Die Herstellung erfolgt nach an sich bekannten Verfahrensweisen, beispielsweise durch Vermischen, Suspendieren oder Lyophilisieren.
Beispiel A: Lyophilisat für die zur Herstellung einer s.c. Injektionslösung 1 mg Verbindung gemäß Beispiel 1 (entsprechend 0,26-0,27 mg Acetatsalz); 0-16,9 Gewichtsteile D-Mannitol, vorzugsweise 0,1-7 Gewichtsteile, jeweils auf Beispiel 1 bezogen, und Wasser für Injektionszwecke (zur Herstellung der Injektionslösung aus dem Lyophilisat).
Herstellung: 1,62 g Beispiel 1 in 30%iger Essigsäure (ca. 1,5 Liter Wasser für Injektionszwecke und 91,17 g Essigsäure) lösen. Die Lösung mit 1,5 Liter Wasser verdünnen. 82,2 g Mannitol zugeben, sterilfiltriren, in sterile 2 ml Injektionsflaschen unter aseptischen Bedingungen abfüllen und gefriertrocknen. Man erhält 1 mg Lyophilisat der Verbindung gemäß Beispiel 1.

Die erfindungsgemäßen Verbindungen sind beispielsweise geeignet zur Behandlung von malignen oder nicht-malignen hormonabhängigen Erkrankungen, wie beispielsweise zur Behandlung des Mammacarcinoms, des Prostatacarcinoms, der Endometriose, des Uterusmyoms, benignen Prostatahyperplasie (BPH) sowie bei der Behandlung von weiblichen oder männlichen Fruchtbarkeitsstörungen, beispielsweise zur Verhinderung eines vorzeitigen Eisprungs bei Patientinnen, die sich einer kontrollierten ovariellen Stimulation, gefolgt von einer Eizellentnehme und Techniken der assistierten Reproduktion unterziehen. Die genannten Behandlungen können bei Säugetieren, insbesondere beim Menschen, durchgeführt werden.

## Patentansprüche

1. LHRH-Antagonisten, **gekennzeichnet durch** die Verbindungen der Formel:
A_{c}-D-Nal(2)¹-D-Cpa²-D-Pal(3)³-Ser⁴-N-Me-Tyr⁵-D-Lys(B)⁶-Nle⁷-Arg⁸-Pro⁹-Sar¹⁰-NH₂
Ac-D-Nal(2)¹-D-Cpa²-D-Pal(3)³-Ser⁴-N-Me-Tyr⁵-D-Lys(B)⁶-Nle⁷-Arg⁸-Pro⁹-D-Ala¹⁰-NH₂
Ac-D-Nal(2)¹-D-Cpa²-D-Pal(3)³-Ser⁴-N-Me-Tyr⁵-D-Lys(B)⁶-Nle⁷-Lys(iPr)⁸-Pro⁹-Sar¹⁰-NH₂
Ac-D-Nal(2)¹-D-Phe(4-Cl)²-D-Pal(3)³-Ser⁴-N-Me-Tyr⁵-D-Lys(B)⁶-Nle⁷-Lys(iPr)⁸-Pro⁹-D-Ala¹⁰-NH₂
sowie deren Salze mit pharmazeutisch akzeptablen Säuren davon.

2. Verbindungen nach Anspruch 1, worin das Salz ein Acetat, Trifluoracetat, oder Embonat ist.

3. Verbindungen nach den Ansprüchen 1 und 2 zur Verwendung als Arzneimittel.

4. Pharmazeutische Zubereitung, umfassend mindestens eine Verbindung nach einem der Ansprüche 1 und 2 sowie übliche Träger- und Hilfsstoffe.

5. Verfahren zur Herstellung von Verbindungen nach Anspruch 1 der allgemeinen Formel I
A-Xxx¹-Xxx²-Xxx³-Xxx⁴Xxx⁵-Xxx⁶-Xxx⁷Xxx⁸-Xxx⁹-Xxx¹⁰-NH₂ (I)
mit A in der Bedeutung Acetyl,
bei dem Fragmente aus mit geeigneten Schutzgruppen versehenen Bausteinen Xxx^{m}, bei denen m eine ganze Zahl von 1 bis 10 bedeutet und Xxx¹ acetyliert ist, an einer Festphase oder in Lösung nach üblichen Verfahren aufgebaut werden, anschließend die Fragmente an einer Festphase durch Segmentkupplung verbunden werden und nach Abschluss der Kupplung die Verbindungen der allgemeinen Formel I mit üblichen Verfahren unter Amidierung am Baustein Xxx¹⁰ von der Festphase abgespalten werden.

6. Verwendung der Verbindungen gemäß den Ansprüchen 1 und 2 zur Herstellung von Arzneimitteln zur Behandlung hormonabhängiger Tumore, insbesondere Prostatacarcinom, Brustkrebs oder Uterusmyom, sowie für nicht-maligne Indikationen, deren Behandlung eine LH-RH-Hormonsuppression erfordert, wie Endometriose, benigne Prostatahyperplasie (BPH) oder zur Behandlung von weiblichen oder männlichen Fruchtbarkeitsstörungen, bei Säugetieren, insbesondere beim Menschen.

7. Verfahren zur Herstellung von pharmazeutischen Zubereitungen gemäß Anspruch 4, wobei man mindestens eine Verbindung gemäß den Ansprüchen 1 und 2 mit den üblichen Träger- und Hilfsstoffen vermischt und als Arzneimittel konfektioniert.

## Claims

1. LHRH antagonists, **characterized by** compounds of the formula
Ac-D-Nal(2)¹-D-Cpa²-D-Pal(3)³-Ser⁴-N-Me-Tyr⁵-D-Lys(B)⁶-Nle⁷-Arg⁸-Pro⁹-Sar¹⁰-NH₂
Ac-D-Nal(2)¹-D-Cpa²-D-Pal(3)³-Ser⁴-N-Me-Tyr⁵-D-Lys(B)⁶-Nle⁷-Arg⁸-Pro⁹-D-Ala¹⁰-NH₂
Ac-D-Nal(2)¹-D-Cpa²-D-Pal(3)³-Ser⁴-N-Me-Tyr⁵-D-Lys(B)⁶-Nle⁷-Lys(iPr)⁸-Pro⁹-Sar¹⁰-NH₂
Ac-D-Nal(2)¹-D-Phe(4-Cl)²-D-Pal(3)³-Ser⁴-N-Me-Tyr⁵-D-Lys(B)⁶-Nle⁷-Lys(iPr)⁸-Pro⁹-D-Ala¹⁰-NH₂
and their salts with pharmaceutically acceptable acids thereof.

2. Compounds according to Claim 1, in which the salt is an acetate, trifluoroacetate or embonate.

3. Compounds according to Claims 1 and 2 for use as medicaments.

4. Pharmaceutical preparation, comprising at least one compound according to one of Claims 1 and 2, and customary vehicles and excipients.

5. Process for the preparation of compounds according to Claim 1 of the general formula I
A-Xxx¹-Xxx²-Xxx³-Xxx⁴-Xxx⁵-Xxx⁶-Xxx⁷-Xxx⁸-Xxx⁹-Xxx¹⁰-NH₂ (I)
with A having the meaning of acetyl,
in which fragments from units Xxx^{m} provided with suitable protective groups, in which m is an integer from 1 to 10 and Xxx¹ is acetylated, are synthesized on a solid phase or in solution according to customary processes, then the fragments are linked to a solid phase by segment coupling and after conclusion of the coupling the compounds of the general formula I are removed from the solid phase using customary processes with amidation on the unit Xxx¹⁰.

6. Use of the compounds according to Claims 1 and 2 for the production of medicaments for the treatment of hormone-dependent tumours, in particular prostate carcinoma, breast cancer or uterine myoma, and for non-malignant indications whose treatment necessitates LH-RH hormone suppression, such as endometriosis, benign prostate hyperplasia (BPH) or for the treatment of female or male fertility disorders, in mammals, in particular in humans.

7. Process for the production of pharmaceutical preparations according to Claim 4, where at least one compound according to Claims 1 and 2 is mixed with the customary vehicles and excipients and formulated as a medicament.

## Revendications

1. Antagonistes de la LHRH
**caractérisé par**
des composés de formule
Ac-D-Nal(2)¹-D-Cpa²-D-Pal(3)³-Ser⁴-N-Me-Tyr⁵-D-Lys(B)⁶-Nle⁷-Arg⁸-Pro⁹-Sar¹⁰-NH₂
Ac-D-Nal(2)¹-D-Cpa²-D-Pal(3)³-Ser⁴-N-Me-Tyr⁵-D-Lys(B)⁶-Nle⁷-Arg⁸-Pro⁹-D-Ala¹⁰-NH₂
Ac-D-Nal(2)¹-D-Cpa²-D-Pal(3)³-Ser-⁴-N-Me-Tyr⁵-D-Lys(B)⁶-Nle⁷-Lys(iPr)⁸-Pro⁹-Sar¹⁰-NH₂
Ac-D-Nal(2)¹-D-Phe(4-Cl)²-D-Pal(3)³-Ser⁴-N-Me-Tyr⁵-D-Lys(B)⁶-Nle⁷-Lys(iPr)⁸-Pro⁹-D-Ala¹⁰-NH₂
ou leurs sels avec des acides pharmacologiquement acceptables.

2. Composés selon la revendication 1 dans lesquels le sel est un acétate, un trifluoroacétate ou un embonate.

3. Composés selon les revendications 1 et 2 pour une utilisation en tant que médicament.

4. Préparation pharmaceutique renfermant au moins un composé selon l'une des revendications 1 et 2 ainsi que des substances support et auxiliaire usuelles.

5. Procédé d'obtention de composés selon la revendication 1 de formule générale I
A-Xxx¹-Xxx²-Xxx³-Xxx⁴-Xxx⁵-Xxx⁶-Xxx⁷-Xxx⁸-Xxx⁹-Xxx¹⁰-XH₂ (I)
dans laquelle A représente un acétyl, selon lequel des fragments de blocs Xxx^{m}, portant des groupes de protection adaptés dans lesquels m représente un nombre entier de 1 à 10 et Xxx¹ est acétylé sont obtenus sur une phase solide ou en solution selon des procédés usuels, puis les fragments sont reliés sur une phase solide par accouplement par segment et, après l'accouplement, les composés de formule générale I sont séparés de la phase solide par des procédés usuels par amidification sur le bloc Xxx¹⁰.

6. Utilisation de composés selon les revendications 1 et 2 pour la préparation de médicaments pour le traitement de tumeurs hormono-dépendantes, en particulier le carcinome de la prostate, le cancer du sein ou le myome de l'utérus, ainsi que pour des indications non malignes dont le traitement nécessite une suppression hormonale de la LH-RH, telles que l'endométriose, l'hyperplasie bénigne de la prostate (BPH) ou pour le traitement de troubles de la fécondité mâle ou femelle chez les mammifères en particulier chez l'homme.

7. Procédé d'obtention de préparations pharmaceutiques selon la revendication 4,
**caractérisé en ce qu'**
on mélange au moins un composé selon les revendications 1 et 2 avec des substances support et auxiliaires usuelles, et on les met sous la forme de médicaments.
